# EUROPEAN PATENT APPLICATION

(11) **EP 4 550 254 A1**
(43) Date of publication of application: **07.05.2025**
(21) Application number: 23306881.6
(22) Date of filing: 30.10.2023
(51) Int. Cl.: G06T 5/73, G06T 7/11, G06T 11/60, A61B 6/12, A61B 6/00, A61B 90/00

(54) **ASSESSING INTERVENTIONAL DEVICE SIZE**

(71) Applicant: Koninklijke Philips N.V., 5656 AG Eindhoven (NL)
(72) Inventor: AUVRAY, Vincent, 5656 Eindhoven (NL); POPOFF, Alexandre, 5656 Eindhoven (NL); COUTEAUX, Vincent, 5656 Eindhoven (NL); ALLAIRE, Stephane, 5656 Eindhoven (NL); LOBANTSEV, Artem, 5656 Eindhoven (NL); OLIVAN BESCOS, Javier, 5656 Eindhoven (NL); SKANDARANi, Youssef, 5656 Eindhoven (NL); HERMANS, Luc, 5656 Eindhoven (NL)
(74) Representative: Philips Intellectual Property & Standards

(57) **Abstract**

A system for assessing a size of a portion of an interventional device, is provided. The system comprising one or more processors configured to: receive X-ray image data comprising a temporal sequence of images representing the interventional device (130) and a reference object (140); identify, in the images in the temporal sequence, at least the portion of the interventional device (130), and the reference object (140); and generate, for a current image in the temporal sequence, a first image representation (150) of the portion of the interventional device (130), and a second image representation (160) of the reference object (140). The first image representation (150) and the second image representation (160) are displayed in relation to each other for comparing the size of the portion of the interventional device (130) with a size of the reference object (140), for example by displaying the image representations in close proximity to each other

## Description

### TECHNICAL FIELD

The present disclosure relates to assessing a size of a portion of an interventional device. A system, an imaging system, a computer-implemented method, and a computer program product, are disclosed.

### BACKGROUND

Temporal sequences of X-ray images are often captured during medical procedures that involve interventional devices. For instance, live temporal sequences of X-ray images are captured in order to navigate interventional devices to a target site in the anatomy, or to execute treatment procedures at the target site. During such medical procedures there is often a need to determine a size of a portion of the interventional device.

By way of an example, balloon angioplasty is a vascular treatment procedure in which an interventional catheter is used to insert an expandable balloon within a stenosed vessel. After being correctly positioned in the stenosed vessel, the expandable balloon of the interventional catheter is expanded, often within a stent, in order to open-up the vessel lumen and to thereby restore blood flow. During this procedure it is useful to be able to verify that the balloon has been expanded to the correct size. If the balloon is under-expanded, blood flow may not be restored to the desired level. Under-expansion of the balloon can also lead to complications such as restenosis, and in-stent thrombosis.

The need to determine the sizes of portions of interventional devices also exists in other types of medical procedures. For instance, atherectomy is a vascular treatment procedure in which deposits are removed from the inner surface of a blood vessel. During atherectomy treatment procedures, a mechanical atherectomy device may be used to scrape the deposits from the inner surface of the vessel. A scraper element of the mechanical atherectomy device may be inserted into the vessel, expanded in order to contact the inner surface of the vessel, and then translated and/or rotated whilst in the expanded state in order to scrape the deposits from the inner surface of the vessel. Here again, it is useful to be able to verify the expanded size of the scraper element in order to provide effective scraping of the vessel's inner surface.

However, it can be challenging to determine the sizes of such portions of interventional devices in temporal sequences of X-ray images. The dimensions of balloons, scraper elements of mechanical atherectomy devices, and also other portions of other types of interventional devices, may only be in the order of a few millimeters. Determining the sizes of such small interventional device features in temporal sequences of X-ray images is challenging due to their small size in the images. Any motion of the interventional device during the temporal sequence, for example due to the beating of the heart, can further hamper the determination of the sizes of such small interventional device features. Consequently, there is a need to determine the sizes of portions of interventional devices in temporal sequences of X-ray images.

### SUMMARY

According to one aspect of the present disclosure, a system for assessing a size of a portion of an interventional device, is provided. The system comprising one or more processors configured to:
receive X-ray image data comprising a temporal sequence of images representing the interventional device and a reference object;
identify, in the images in the temporal sequence, at least the portion of the interventional device, and the reference object;
generate, for a current image in the temporal sequence, a first image representation of the portion of the interventional device, and a second image representation of the reference object; and
display the first image representation and the second image representation such that the portion of the interventional device, and the reference object, in relation to each other for comparing the size of the portion of the interventional device with a size of the reference object.

Accordingly, the system facilitates a physician to make a comparative assessment of the size of the portion of the interventional device in relation to the size of the reference object. This comparison is readily made by a user because the reference object has a known size and the physician is familiar with its size. Consequently, the system facilitates an assessment of the size of the portion of the interventional device whilst overcoming the drawback of its small size in the images.

In certain examples, the first and second image representations are displayed such that the portion of the interventional device and the reference object are shown closer to one another than in the temporal sequence of images. For example, the portion of the interventional device and the reference object may be shown in close proximity to each other.

Further aspects, features, and advantages of the present disclosure will become apparent from the following description of examples, which is made with reference to the accompanying drawings.

### BRIEF DESCRIPTION OF THE DRAWINGS

Fig. 1 is a schematic diagram illustrating an example of a system 100 for assessing a size of a portion of an interventional device, in accordance with some aspects of the present disclosure.
Fig. 2 is a flowchart illustrating an example of a computer-implemented method of assessing a size of a portion of an interventional device, in accordance with some aspects of the present disclosure.
Fig. 3 is an example of a temporal sequence of images 120 representing an interventional device 130 and a reference object 140, in accordance with some aspects of the present disclosure.
Fig. 4 is an example of a first image representation 150 of a portion of an interventional device 130 and a second image representation 160 of a reference object 140 wherein the portion of the interventional device 130 and the reference object 140 are displayed relatively closer to one another than in the temporal sequence of images 120 for comparing a size of the portion of the interventional device 130 with a size of the reference object 140, in accordance with some aspects of the present disclosure.
Fig. 5 is a schematic diagram illustrating an example of a method of assessing a size of a portion of an interventional device, in accordance with some aspects of the present disclosure.
Fig. 6 is an example of a first image representation 150 of a portion of an interventional device 130 and a second image representation 160 of a reference object 140 wherein the orientation of the interventional device 130 in the first image representation has been adjusted to correspond to the orientation of the reference object 140 in the second image representation 160, in accordance with some aspects of the present disclosure.
Fig. 7 is an example of a first image representation 150 of a portion of an interventional device 130 and a second image representation 160 of a reference object 140 including a reference scale 190, in accordance with some aspects of the present disclosure.

### DETAILED DESCRIPTION

Examples of the present disclosure are provided with reference to the following description and Figures. In this description, for the purposes of explanation, numerous specific details of certain examples are set forth. Reference in the specification to "an example", "an implementation" or similar language means that a feature, structure, or characteristic described in connection with the example is included in at least that one example. It is also to be appreciated that features described in relation to one example may also be used in another example, and that all features are not necessarily duplicated in each example for the sake of brevity. For instance, features described in relation to a system, may be implemented in a computer-implemented method, and in a computer program product, in a corresponding manner.

In the following description, reference is made to examples of systems and methods that facilitate an assessment of a size of a portion of an interventional device. In some examples, the interventional device is an interventional catheter, and the size that is determined is a diameter/ width of a balloon of the interventional catheter. In some examples, reference is made to an assessment of the diameter/ width of the balloon of the interventional catheter that is facilitated by displaying the balloon of the interventional catheter, and a reference object, and in which the reference object is an injection catheter. However, it is to be appreciated that the interventional catheter, the balloon, the injection catheter, and also the diameter/ width serve only as an examples. Thus, the interventional device may alternatively be another interventional device than an interventional catheter, the portion of the interventional device may alternatively be another portion of an interventional device than a balloon, the reference object may alternatively be another reference object than an injection catheter, and the size that is assessed may be another dimension than a diameter/ width. By way of some further examples, the interventional device may be a mechanical atherectomy device, the injection catheter may alternatively be another type of catheter, and the portion of the interventional device may be an expandable portion of the interventional device, or a distal end of the interventional device. Thus, in some examples, the interventional device is an intravascular interventional device. In some examples, the reference object is an intravascular reference object.

It is noted that the computer-implemented methods disclosed herein may be provided as a non-transitory computer-readable storage medium including computer-readable instructions stored thereon, which, when executed by at least one processor, cause the at least one processor to perform the method. In other words, the computer-implemented methods may be implemented in a computer program product. The computer program product can be provided by dedicated hardware, or hardware capable of running the software in association with appropriate software. When provided by a processor, the functions of the method features can be provided by a single dedicated processor, or by a single shared processor, or by a plurality of individual processors, some of which can be shared. The functions of one or more of the method features may for instance be provided by processors that are shared within a networked processing architecture such as a client/server architecture, a peer-to-peer architecture, the Internet, or the Cloud.

The explicit use of the terms "processor" or "controller" should not be interpreted as exclusively referring to hardware capable of running software, and can implicitly include, but is not limited to, digital signal processor "DSP" hardware, read only memory "ROM" for storing software, random access memory "RAM", a non-volatile storage device, and the like. Furthermore, examples of the present disclosure can take the form of a computer program product accessible from a computer-usable storage medium, or a computer-readable storage medium, the computer program product providing program code for use by or in connection with a computer or any instruction execution system. For the purposes of this description, a computer-usable storage medium or a computer readable storage medium can be any apparatus that can comprise, store, communicate, propagate, or transport a program for use by or in connection with an instruction execution system, apparatus, or device. The medium can be an electronic, magnetic, optical, electromagnetic, infrared, or a semiconductor system or device or propagation medium. Examples of computer-readable media include semiconductor or solid-state memories, magnetic tape, removable computer disks, random access memory "RAM", read-only memory "ROM", rigid magnetic disks and optical disks. Current examples of optical disks include compact disk-read only memory "CD-ROM", compact disk-read/write "CD-R/W", Blu-Ray^{™} and DVD.

It is also noted that some operations that are described as being performed by the one or more processors of the system disclosed herein may be implemented using artificial intelligence techniques. Suitable techniques may include machine learning techniques, as well as deep learning techniques such as neural networks. For instance, one or more neural networks, may be trained in a supervised, or in some cases unsupervised, manner, to implement the operations that are performed by the one or more processors.

As mentioned above, there is a need to determine the sizes of portions of interventional devices in temporal sequences of X-ray images.

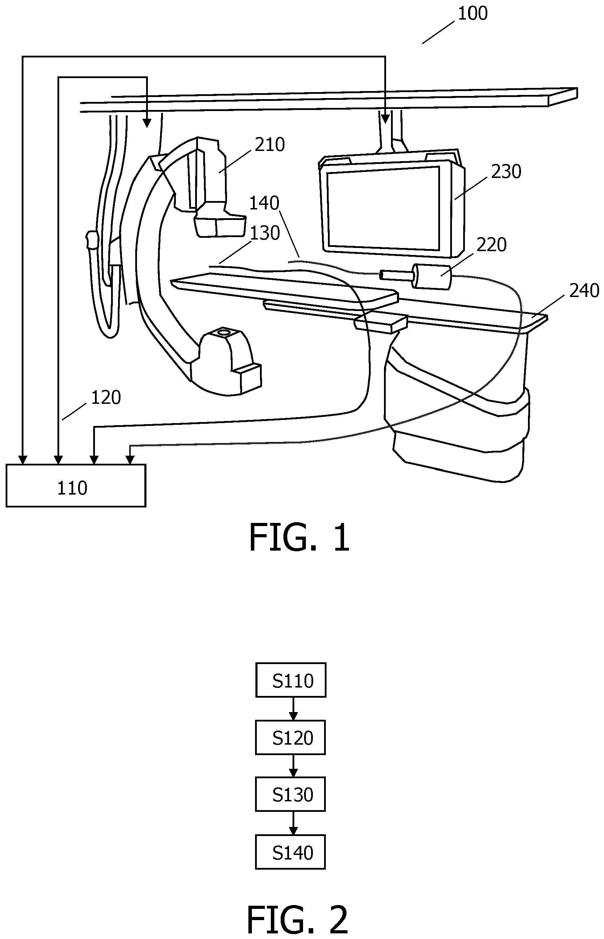

Fig. 1 is a schematic diagram illustrating an example of a system 100 for assessing a size of a portion of an interventional device, in accordance with some aspects of the present disclosure. Fig. 2 is a flowchart illustrating an example of a computer-implemented method of assessing a size of a portion of an interventional device, in accordance with some aspects of the present disclosure. It is noted that operations described as being performed by the one or more processors 110 of the system 100 illustrated in Fig. 1, may also be performed in the method illustrated in Fig. 2. Likewise, operations described in relation to the method described with reference to Fig. 2 may also be performed by the one or more processors 110 of the system 100 illustrated in Fig. 1. With reference to Fig. 1, and Fig. 2, the system 100 for assessing a size of a portion of an interventional device, comprises one or more processors 110 configured to:
receive S110 X-ray image data comprising a temporal sequence of images 120 representing the interventional device 130 and a reference object 140;
identify S120, in the images in the temporal sequence, at least the portion of the interventional device 130, and the reference object 140;
generate S130, for a current image 120' in the temporal sequence, a first image representation 150 of the portion of the interventional device 130, and a second image representation 160 of the reference object 140; and
display S140 the first image representation 150 and the second image representation 160 in relation to each other for comparing the size of the portion of the interventional device 130 with a size of the reference object 140.

Thus the system facilitates a comparative assessment of the size of the portion of the interventional device in relation to the size of the reference object. This comparison is readily made by a user, and consequently the system facilitates an assessment of the size of the portion of the interventional device whilst overcoming the drawback of its small size in the images. For example, the system displays the first image representation and the second image representation such that the portion of the interventional device and the reference object are displayed relatively closer to one another than in the temporal sequence of image.

The operations that are performed by the one or more processors of the system 100 are described in more detail below.

In the operation S110, X-ray image data is received. The X-ray image data comprises a temporal sequence of images 120 representing the interventional device 130 and a reference object 140.

The X-ray image data that is received in the operation S 110 may be generated by various types of X-ray imaging systems. These include projection X-ray imaging systems, computed tomography "CT" imaging systems, spectral X-ray projection imaging systems, and spectral CT imaging systems. Spectral X-ray projection imaging systems, and spectral CT imaging systems generate X-ray attenuation data representing X-ray attenuation within multiple different energy intervals. This is in contrast to conventional projection X-ray imaging systems, and conventional CT imaging systems, and which generate X-ray attenuation data representing X-ray attenuation within a single energy interval. The X-ray attenuation data generated by such spectral imaging systems may be processed, e.g. using various material decomposition algorithms, in order to distinguish between media that have similar X-ray attenuation values when measured within a single energy interval, and which would be indistinguishable in X-ray attenuation data obtained using conventional projection X-ray imaging systems, or conventional CT imaging systems. Thus, the X-ray attenuation data generated by spectral X-ray projection imaging systems, or by spectral CT imaging systems, may be used to provide images with improved specificity to materials such as tissue, bone, and so forth.

With continued reference to the operation S110, the X-ray image data that is received in the operation S110 may be received from various sources, including from a medical imaging system, such as the imaging systems described above, or from another source, such as a computer readable storage medium, the internet, the cloud, and so forth. The sequence of angiographic images 120 may be received via any form of data communication, including wired and wireless communication. By way of some examples, when wired communication is used, the communication may take place via an electrical or optical cable, and when wireless communication is used, the communication may for example be via RF or infrared signals.

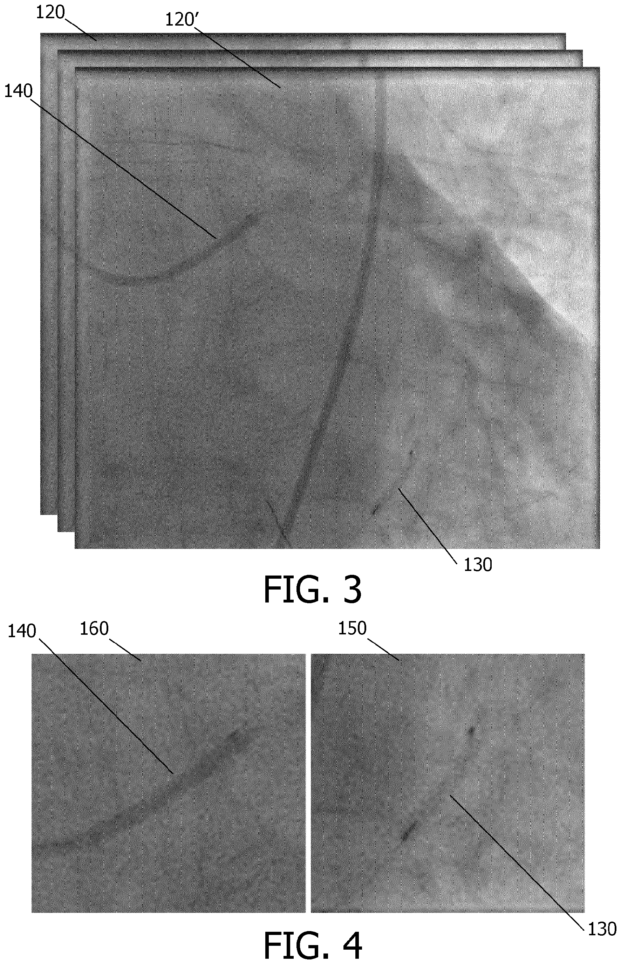

With continued reference to the operation S110, the temporal sequence of images 120 represents an interventional device 130 and a reference object 140. Fig. 3 is an example of a temporal sequence of images 120 representing an interventional device 130 and a reference object 140, in accordance with some aspects of the present disclosure. In the example illustrated in Fig. 3, the interventional device 130 is an interventional catheter, and the reference object 140 is an injection catheter. The interventional catheter includes a balloon that is located towards the distal end of the interventional catheter in Fig. 3. The interventional catheter is used to introduce the balloon to the vasculature.

In the example illustrate in Fig. 3, the balloon may be navigated along the interventional catheter to a target site within stenosed vessel, and subsequently expanded in order to open-up the vessel lumen and to thereby restore blood flow. During this procedure, the injection catheter 130 may be used to intermittently inject contrast agent into the vasculature in order to provide visibility of the vessels in the images. In contrast to the injection catheter, the interventional catheter tends to be relatively stiffer. The visibility of the vessels that is provided by the contrast agent injection assists a physician in tasks such as navigating the balloon to the target site. The contrast agent may be injected using a contrast injector, often referred to simply as an injector, such as the injector 220 illustrated in Fig. 1, for example. During this procedure it is useful to be able to verify that the balloon has been expanded to the correct size. However, as may be appreciated from the temporal sequence of images 120 illustrated in Fig. 3, the balloon of the interventional catheter has a small diameter/ width in the images. Consequently it can be difficult to measure the diameter/ width of the balloon accurately.

Referring now to the operation S120 mentioned above with reference to Fig. 1 and Fig. 2, in this operation, at least the portion of the interventional device 130, and the reference object 140, are identified in the images in the temporal sequence. Thus, with reference to the example in Fig. 3, in this operation, at least the balloon, and the injection catheter, are identified in the images in the temporal sequence. In this operation, other elements of the interventional device may also be identified. For instance, if the portion of the interventional device that is identified is a balloon of an interventional catheter, then other element(s) of the interventional catheter, such as a distal end of the interventional catheter, may also be identified.

Various techniques may be used to perform the operation S120. For instance, one technique includes using an artificial intelligence "AI" algorithm that is trained to identify the associated regions in the images in the temporal sequence. An example of such an AI algorithm is disclosed in a document by Jiang, P., et al., "A review of Yolo algorithm developments", Procedia Computer Science, Vol. 199, 2022, pages 1066 - 1073. Another technique includes using segmentation to identify these features in the images in the temporal sequence. An example of such a segmentation technique is disclosed in a document by Ronneberger, O., et al., "U-Net: Convolutional Networks for Biomedical Image Segmentation", In: Navab, N., Hornegger, J., Wells, W., Frangi, A. (Eds.) Medical Image Computing and Computer-Assisted Intervention - MICCAI 2015. MICCAI 2015. Lecture Notes in Computer Science, Vol. 9351. It is noted that neither technique requires an accurate delineation of the at least the portion of the interventional device 130, and the reference object 140 since the goal of the operation S120 is simply to identify the respective devices and their locations rather than to provide absolute measurements of their sizes.

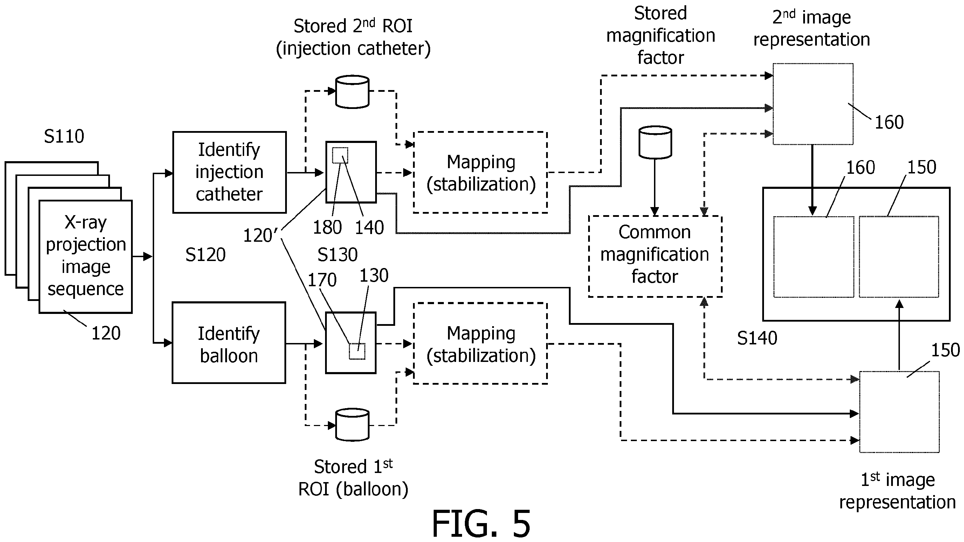

The operation of identifying S120, in the images in the temporal sequence, at least the portion of the interventional device 130, and the reference object 140, is also illustrated in Fig. 5, which is a schematic diagram illustrating an example of a method of assessing a size of a portion of an interventional device, in accordance with some aspects of the present disclosure. The operations S110, and S120, illustrated in Fig. 5 correspond to those described above with reference to Fig. 1 and Fig. 2 in the case that the portion of the interventional device 130 is a balloon of an interventional catheter, and the reference object 140 is an injection catheter. In Fig. 5, the operation S120 of identifying the at least the portion of the interventional device 130, and the reference object 140, is illustrated in the central portion of Fig. 5; i.e. "identify injection catheter" and "identify balloon".

Referring now to the operation S130 mentioned above with reference to Fig. 1 and Fig. 2, in this operation, for a current image 120' in the temporal sequence, a first image representation 150 of the portion of the interventional device 130 is generated, and a second image representation 160 of the reference object 140 is generated.

The image representations may be generated in various ways. In one example, the one or more processors 110 are configured to define, in the images in the temporal sequence, a first region of interest 170 surrounding the portion of the interventional device 130 and/or a second region of interest 180 surrounding the reference object 140. In this example, an extent of the first image representation 150 corresponds to the first region of interest 170 and/or an extent of the second image representation 160 corresponds to the second region of interest 180, respectively. The regions of interest may be defined in various ways, such as by cropping the regions of interest to fixed shapes that are centered on a point defined on the portion of the interventional device 130, and a point defined on the reference object 140, respectively. The point may be a distal end of the portion of the interventional device 130, and a distal end of the reference object 140, for example.

The generation of the first image representation 150 and the second image representation 160 in accordance with this example, i.e. by defining such regions of interest, is illustrated in the central portion of Fig. 5. Thus, in the central portion of Fig. 5 that follows the identification of the injection catheter and the balloon, the upper and lower branches illustrate the identification of a first region of interest 170 and a second region of interest, in a current image 120'. The resulting first image representation 150 and second image representation 160 are illustrated in the upper and lower right-hand side of Fig. 5. Examples of these image representations are also illustrated in Fig. 4, which is an example of a first image representation 150 of a portion of an interventional device 130 and a second image representation 160 of a reference object 140 wherein the portion of the interventional device 130 and the reference object 140 are displayed relatively closer to one another than in the temporal sequence of images 120 for comparing a size of the portion of the interventional device 130 with a size of the reference object 140, in accordance with some aspects of the present disclosure.

Referring now to the operation S140 mentioned above with reference to Fig. 1 and Fig. 2, in this operation, the first image representation 150 and the second image representation 160 are displayed in relation to each other or comparing the size of the portion of the interventional device 130 with a size of the reference object 140. For example, as shown in Fig. 4, the portion of the interventional device 130 and the reference object 140 are displayed in relative close proximity to one another, closer than in the original temporal sequence of images 120.

The first image representation 150 and the second image representation 160 may be updated continuously for consecutive current images in the temporal sequence. This provides a real-time comparison of the size of the portion of the interventional device 130 with the size of the reference object 140.

Exemplary arrangements of the image representations of the portion of the interventional device and the reference object include displaying the portion of the interventional device, and the reference object, adjacent to one another, or side by side. As may be observed in Fig. 4, displaying first image representation 150 and the second image representation 160 in this manner facilitates a comparison of the size of the portion of the interventional device 130 with a size of the reference object 140. In general, the size may refer to any dimension of the portion of the interventional device 130, and the reference object 140, including for example a diameter, or a width, or a length, or another dimension. Such a comparison would be difficult to make in the temporal sequence of images 120 because the portion of the interventional device 130 and the reference object 140 have a relatively larger separation.

Still, other arrangements of the relative image representations facilitating a size comparison between the objects, including but not limited to overlays of the reference object or a schematic representation thereof on top of the portion of the interventional device, or vice versa, may be envisaged herein.

The operation S140 is also illustrated schematically in the central right-hand portion of Fig. 5. Here, the portion of the interventional device, and the reference object, are likewise displayed relatively closer to one another than in the temporal sequence of images 120 in order to facilitate a comparison of their sizes.

Instead of generating the first image representation 150 of the portion of the interventional device 130, and the second image representation 160 of the reference object 140, by defining a region of interest surrounding each of the portion of the interventional device 130, and the reference object 140, as was described above with reference to Fig. 4 and Fig. 5, the first image representation 150 and the second image representation 160 may alternatively be generated in other ways. For example, a single region of interest may be defined in the images in the temporal sequence that includes the portion of the interventional device 130, or the reference object 140. This region of interest may then be cropped, using the techniques described above, and then overlaid onto the current image 120' relatively closer to the other of the portion of the interventional device 130, and the reference object 140 such that the portion of the interventional device, and the reference object, are displayed relatively closer to one another than in the temporal sequence of images for comparing the size of the portion of the interventional device 130 with a size of the reference object 140.

Thus, with reference to Fig. 3, since the system 100 displays the first image representation and the second image representation such that the balloon, and the injection catheter are displayed relatively closer to one another than in the temporal sequence of images, the system facilitates a physician to make a comparative assessment of the size of the balloon in relation to the size of the injection catheter. This comparison is readily made by a user because the injection catheter has a known size, and the physician is familiar with its size. The diameter of the injection catheter may be known to be 2 millimeters, for example. Consequently, the system facilitates an assessment of the size of the balloon whilst overcoming the drawback of its small size in the images.

As mentioned above, other types of interventional devices, and other types of objects, may alternatively serve as the interventional device, and the reference object, respectively. For instance, the interventional device may be a mechanical atherectomy device, and the reference object may in general be any object for which a physician knows the size. The reference object may have a similar size to the portion of the interventional device. The interventional device and the reference object may both lie at the same depth with respect to X-ray source of the X-ray imaging system that generates the temporal sequence of images.

As mentioned above, a further factor that can hamper the determination of a size of features of an interventional device in temporal sequences of X-ray images, is motion of the interventional device during the temporal sequence. For instance, in the example illustrated in Fig. 3, the beating of the heart can cause significant motion of the balloon of the interventional catheter 130 during the temporal sequence of images 120. The present disclosure provides various techniques to assist in the assessment of the size of the portion of the interventional device in the presence of such cardiac motion, and also other sources of motion.

In one example, the one or more processors 110 are configured to generate S130 the first image representation 150 for the current image 120' in the temporal sequence by:
mapping the portion of the interventional device 130 in the current image 120' in the temporal sequence, to a position of the portion of the interventional device in the first image representation 150 generated for an earlier image in the temporal sequence.

The effect of this mapping is to reduce the motion of the portion of the interventional device in the first image representation, or in other words to stabilize its motion. With reference to the example illustrated in Fig. 4, in this example it stabilizes the motion of the balloon. This, in turn facilitates a more accurate comparison of the size of the balloon with the size of the injection catheter. This mapping operation is illustrated in Fig. 5 by way of the lower Mapping (stabilization) label within the dashed box in the center of Fig. 5.

The mapping that is performed in this example may be performed using various techniques, including for example by mapping a position of a feature, e.g. a centroid, or a distal end, of the portion of the interventional device 130 in the current image 120' in the temporal sequence, to a position of the feature in the first image representation 150 generated for an earlier image in the temporal sequence.

In one example, the operation of mapping the portion of the interventional device 130 in the current image 120' in the temporal sequence, to a position of the portion of the interventional device in the first image representation 150 generated for an earlier image in the temporal sequence, comprises rigidly registering the portion of the interventional device 130 in the current image 120' in the temporal sequence, to the portion of the interventional device 130 in the first image representation 150 generated for the earlier image in the temporal sequence.

The rigid registration that is provided by this example reduces the amount of motion of the portion of the interventional device between consecutive images. It also facilitates an accurate representation in the first image representation 150, of the shape, and the size, of the portion of the interventional device in the live sequence. This is useful in e.g. assessing the size of a balloon because its size, and shape, change during its expansion.

In another example, similar mapping operations are performed on the reference object to provide the second image representation 160. Thus, in one example, the one or more processors 110 are configured to generate S130 the second image representation 160 for the current image 120' in the temporal sequence by:
mapping the reference object 140 in the current image in the temporal sequence, to a position of the reference object in the second image representation 160 generated for an earlier image in the temporal sequence.

The effect of this mapping is to reduce the motion of the reference object in the second image representation, or in other words to stabilize the motion of the reference object. This mapping operation is illustrated in Fig. 5 by way of the upper Mapping (stabilization) label within the dashed box in the center of Fig. 5.

In a related example, the operation of mapping the reference object 140 in the current image 120' in the temporal sequence, to a position of the reference object in the second image representation 160 generated for an earlier image in the temporal sequence, comprises rigidly registering the reference object in the current image 120' in the temporal sequence, to the reference object in the second image representation 160 generated for the earlier image in the temporal sequence.

These mapping operations provide similar benefits to the stability of the reference object as the mappings that are performed for the portion of the interventional device. However, it is noted that in some situations, their impact on the assessment of the size of the portion of the interventional device may be relatively lower. For instance, if the reference object is located in a relatively more stable portion of the anatomy, then the benefit of performing the mapping operations on the reference object may be relatively lower. For example, in the case of a coronary angioplasty for the treatment of a stenosis in a coronary artery, contrast agent injection is often injected using an injection catheter that is located in the aortic arch whilst the balloon is located in the coronary artery. The motion of the injection catheter in this position is typically relatively lower than the motion of a balloon in the coronary arteries. Thus, in this case, the benefit of performing the mapping is relatively lower for the balloon than for the injection catheter.

In another example, the one or more processors 110 are configured to magnify, by a same magnification factor, each of the first image representation 150 and the second image representation 160.

The magnification that is provided by this example facilitates an easier comparison between the size of the portion of the interventional device and the size of the reference object. This magnification operation is illustrated in Fig. 5 by way of the Common magnification label towards the right-hand side of Fig. 5. The magnification factor may be selected so as to fit the largest of the first region of interest 170 and the second region of interest 180 over the display, for example.

In a related example, the one or more processors 110 are configured to adapt the magnification factor throughout the temporal sequence based on a size of the portion of the interventional device 130 in the first image representation 150, or based on a size of the reference object 140 in the second image representation 160.

In another related example, the one or more processors 110 are configured to smooth the adaptation of the magnification factor over time.

The effect of this adaptation is to avoid abrupt changes in the size of the portion of the interventional device and the reference object over time. The magnification factor may be adapted by applying a filter to the size of the portion of the interventional device 130 in the first image representation 150, or to the size of the reference object 140 in the second image representation 160, in the calculation of the magnification factor. The filter may be a Gaussian filter, or a Kalman filter, for example.

In another example, the one or more processors 110 are configured to adjust an orientation of the first image representation 150 and/or the second image representation 160 such that an orientation of the interventional device 130 in the first image representation corresponds to an orientation of the reference object 140 in the second image representation 160.

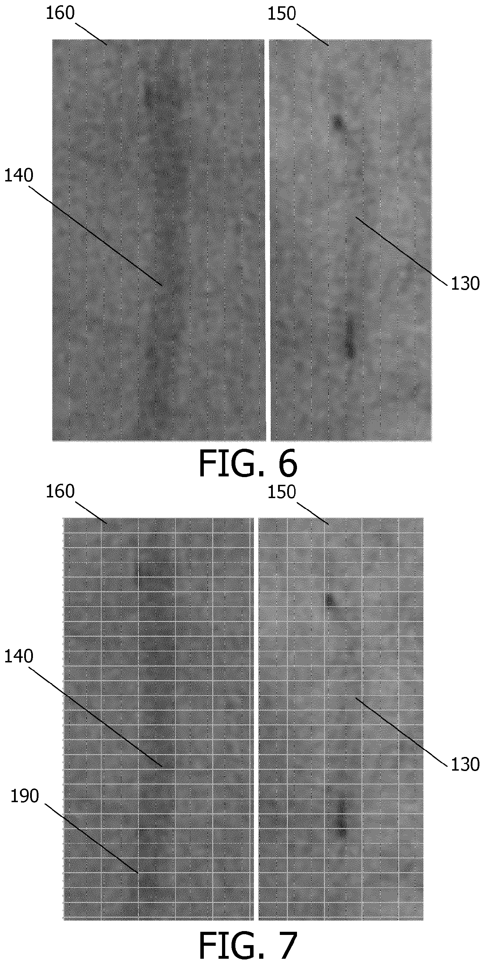

Fig. 6 is an example of a first image representation 150 of a portion of an interventional device 130 and a second image representation 160 of a reference object 140 wherein the orientation of the interventional device 130 in the first image representation has been adjusted to correspond to the orientation of the reference object 140 in the second image representation 160, in accordance with some aspects of the present disclosure. As may be appreciated from Fig. 6, adjusting the orientation in accordance with this example, makes it easier to compare the size of the portion of the interventional device 130 with a size of the reference object 140. The orientations that are brought into correspondence may be an orientation of a longitudinal axis of the portion of the interventional device 130 and a longitudinal axis of the reference object 140, for example. These orientations may be determined, and adjusted, using image processing techniques.

In another example, the one or more processors 110 are configured to display a third image representation comprising a second reference object. The third image representation and the first image representation 150 are displayed such that the portion of the interventional device and the second reference object are displayed relatively closer to one another than in the temporal sequence of images 120 for comparing the size of the portion of the interventional device 130 with a size of the second reference object.

In this example, a further, second reference object is displayed. The second reference object may be displayed in a similar manner to the reference object, e.g. by defining, in the images in the temporal sequence, a region of interest surrounding the second reference object, and displaying this side-by-side, i.e. adjacent to, the first image representation 150. The display of the second reference object in this manner provides a further reference size for use in assessing the size of the portion of the interventional device.

The second reference object may in general be any object for which a physician knows the size. The second reference object may have a similar size to the portion of the interventional device. The interventional device and the second reference object may both lie at the same depth with respect to X-ray source of the X-ray imaging system that generates the temporal sequence of images. The second reference object may be a different object to the reference object. For instance, the second reference object may be a probe, a pacemaker, a guidewire, and so forth.

In another example, the one or more processors 110 are configured to display a reference scale 190 in the first image representation 150 and the second image representation 160.

This example is illustrated in Fig. 7, which is an example of a first image representation 150 of a portion of an interventional device 130 and a second image representation 160 of a reference object 140 including a reference scale 190, in accordance with some aspects of the present disclosure. The reference scale may be displayed in the form of a grid, as illustrated in Fig. 7. The reference scale eases the assessment of the size of the portion of the interventional device in comparison to the size of the reference object. The intervals in the reference scale may be derived from the size of the portion of the interventional device. For example, a periodicity of the grid may be defined as a multiple, or a fraction, of the diameter/ width of the portion of the interventional device.

In another example, the one or more processors 110 are configured to freeze the display of the first image representation 150 and/or the second image representation 160. Freezing either of these image representations eases the assessment of the size of the portion of the interventional device in comparison to the size of the reference object because it eliminates its motion. In one example, the size of the display of the first image representation 150 is frozen whilst the second image representation 160 is continually updated for subsequent current images in the temporal sequence. This eliminates motion of the reference object, the size of which is not expected to change, whilst providing for the continuous updating of the size of the portion of the interventional device. This example may be useful in e.g. balloon angioplasty procedures, and wherein the size of the reference catheter does not change over time, whereas the size of balloon changes during its expansion.

It is noted that the system 100 described above may also include additional features for use in relation to the operations performed by the one or more processors 110. For instance, the system 100 may include one or more of: an X-ray imaging system such as the projection X-ray imaging system 210 illustrated in Fig. 1 for generating the X-ray image data; an injector 220 for injecting a contrast agent into the vasculature of a subject; a monitor 230 for displaying the temporal sequence of images 120, the first image representation 150 and the second image representation 160, and other outputs provided by the one or more processors 110; a patient bed 240; and a user input device configured to receive user input (not illustrated in Fig. 1), such as a keyboard, a mouse, a touchscreen, and so forth.

In another example, an imaging system 210 for assessing a size of a portion of an interventional device, is provided. The imaging system comprises one or more processors 110 configured to:
receive S110 X-ray image data comprising a temporal sequence of images 120 representing the interventional device 130 and a reference object 140;
identify S120, in the images in the temporal sequence, at least the portion of the interventional device 130, and the reference object 140;
generate S130, for a current image 120' in the temporal sequence, a first image representation 150 of the portion of the interventional device 130, and a second image representation 160 of the reference object 140; and
display S140 the first image representation 150 and the second image representation 160 in relation to each other for comparing the size of the portion of the interventional device 130 with a size of the reference object 140.

In another example, a computer-implemented method of assessing a size of a portion of an interventional device, is provided. The method comprises:
receiving S 110 X-ray image data comprising a temporal sequence of images 120 representing the interventional device 130 and a reference object 140;
identifying S120, in the images in the temporal sequence, at least the portion of the interventional device 130, and the reference object 140;
generating S130, for a current image 120' in the temporal sequence, a first image representation 150 of the portion of the interventional device 130, and a second image representation 160 of the reference object 140; and
displaying S140 the first image representation 150 and the second image representation 160 such that the portion of the interventional device, and the reference object, are displayed relatively closer to one another than in the temporal sequence of images 120 for comparing the size of the portion of the interventional device 130 with a size of the reference object 140.

In another example, a computer program product is provided. The computer program product comprises instructions which when executed by one or more processors 110, cause the one or more processors to carry out a method of assessing a size of a portion of an interventional device. The method comprises:
receiving S 110 X-ray image data comprising a temporal sequence of images 120 representing the interventional device 130 and a reference object 140;
identifying S120, in the images in the temporal sequence, at least the portion of the interventional device 130, and the reference object 140;
generating S130, for a current image 120' in the temporal sequence, a first image representation 150 of the portion of the interventional device 130, and a second image representation 160 of the reference object 140; and
displaying S140 the first image representation 150 and the second image representation 160 such that the portion of the interventional device, and the reference object, are displayed relatively closer to one another than in the temporal sequence of images 120 for comparing the size of the portion of the interventional device 130 with a size of the reference object 140.

The above examples are to be understood as illustrative of the present disclosure, and not restrictive. Further examples are also contemplated. For instance, the examples described in relation to a system, may also be provided by the computer-implemented method, or by the computer program product, or by a computer-readable storage medium, in a corresponding manner. It is to be understood that a feature described in relation to any one example may be used alone, or in combination with other described features, and may be used in combination with one or more features of another of the examples, or a combination of other examples. Furthermore, equivalents and modifications not described above may also be employed without departing from the scope of the invention, which is defined in the accompanying claims. In the claims, the word "comprising" does not exclude other elements or operations, and the indefinite article "a" or "an" does not exclude a plurality. The mere fact that certain features are recited in mutually different dependent claims does not indicate that a combination of these features cannot be used to advantage. Any reference signs in the claims should not be construed as limiting their scope.

## Claims

1. A system (100) for assessing a size of a portion of an interventional device, the system comprising one or more processors (110) configured to:
receive (S110) X-ray image data comprising a temporal sequence of images (120) representing the interventional device (130) and a reference object (140);
identify (S120), in the images in the temporal sequence, at least the portion of the interventional device (130), and the reference object (140);
generate (S130), for a current image (120') in the temporal sequence, a first image representation (150) of the portion of the interventional device (130), and a second image representation (160) of the reference object (140); and
display (S140) the first image representation (150) and the second image representation (160) in relation to each other for comparing the size of the portion of the interventional device (130) with a size of the reference object (140).

2. The system of claim 1, wherein the first image representation and the second image representation are displayed such that the portion of the interventional device and the reference object are closer to one another than in the temporal sequence of images (120).

3. The system according to claim 1 or 2, wherein the one or more processors (110) are further configured to define, in the images in the temporal sequence, a first region of interest (170) surrounding the portion of the interventional device (130) and/or a second region of interest (180) surrounding the reference object (140); and
wherein an extent of the first image representation (150) corresponds to the first region of interest (170) and/or an extent of the second image representation (160) corresponds to the second region of interest (180), respectively.

4. The system according to any preceding claim, wherein the one or more processors (110) are configured to generate (S130) the first image representation (150) for the current image (120') in the temporal sequence by:
mapping the portion of the interventional device (130) in the current image (120') in the temporal sequence to a position of the portion of the interventional device in the first image representation (150) generated for an earlier image in the temporal sequence.

5. The system according to claim 4, wherein mapping the portion of the interventional device (130) comprises rigidly registering the portion of the interventional device (130) in the current image (120') in the temporal sequence, to the portion of the interventional device (130) in the first image representation (150) generated for the earlier image in the temporal sequence.

6. The system according to any previous claim, wherein the one or more processors (110) are configured to generate (S130) the second image representation (160) for the current image (120') in the temporal sequence by:
mapping the reference object (140) in the current image in the temporal sequence, to a position of the reference object in the second image representation (160) generated for an earlier image in the temporal sequence.

7. The system according to claim 6, wherein mapping the reference object (140) comprises rigidly registering the reference object in the current image (120') in the temporal sequence, to the reference object in the second image representation (160) generated for the earlier image in the temporal sequence.

8. The system according to any previous claim, wherein the one or more processors (110) are further configured to magnify, by a same magnification factor, each of the first image representation (150) and the second image representation (160).

9. The system according to claim 8, wherein the one or more processors (110) are further configured to adapt the magnification factor throughout the temporal sequence based on the size of the portion of the interventional device (130) in the first image representation (150), or based on a size of the reference object (140) in the second image representation (160).

10. The system according to claim 9, wherein the one or more processors (110) are further configured to smooth the adaptation of the magnification factor over time.

11. The system according to any previous claim, wherein the one or more processors (110) are further configured to adjust an orientation of the first image representation (150) and/or the second image representation (160) such that an orientation of the interventional device (130) in the first image representation corresponds to an orientation of the reference object (140) in the second image representation (160).

12. The system according to any previous claim, wherein the one or more processors (110) are further configured to display a reference scale (190) in the first image representation (150) and the second image representation (160).

13. The system according to any previous claim, wherein the one or more processors (110) are further configured to freeze the display of the first image representation (150) and/or the second image representation (160).

14. A computer-implemented method of assessing a size of a portion of an interventional device, the method comprising:
receiving (S110) X-ray image data comprising a temporal sequence of images (120) representing the interventional device (130) and a reference object (140);
identifying (S120), in the images in the temporal sequence, at least the portion of the interventional device (130), and the reference object (140);
generating (S130), for a current image (120') in the temporal sequence, a first image representation (150) of the portion of the interventional device (130), and a second image representation (160) of the reference object (140); and
displaying (S140) the first image representation (150) and the second image representation (160) in relation to each other for comparing the size of the portion of the interventional device (130) with a size of the reference object (140).

15. A computer program product comprising instructions which when executed by one or more processors (110), cause the one or more processors to carry out the method according to claim 14.
